# EUROPEAN PATENT APPLICATION

(11) **EP 2 887 050 A1**
(43) Date of publication of application: **24.06.2015**
(21) Application number: 13199357.8
(22) Date of filing: 23.12.2013
(51) Int. Cl.: G01N 21/31, A61B 5/00

(54) **Method for marker-free demarcation of tissues**

(71) Applicant: Hochschule Reutlingen, 72762 Reutlingen (DE)
(72) Inventor: Kessler, Rudolf W., 72762 Reutlingen (DE); Ostertag, Edwin, 72762 Reutlingen (DE); Ritz, Rainer, 35043 Marburg (DE); Bartsch, Jörg-Walter, 35041 Marburg (DE)
(74) Representative: Elbel, Michaela

(57) **Abstract**

The invention relates to a method for a marker-free demarcation of distinct areas of a tissue *in vitro,* comprising the steps of recording at least two different spectra and/or spectral images of the tissue, analyzing the recorded spectra and/or spectral images by a multivariate data analysis to segment the tissues into distinct areas of similar spectral signature, and classifying each area as physiological, pathological or dead according to its spectral signature.

## Description

### Field of the invention

The invention relates to a method for a marker-free demarcation of distinct areas of a tissue *in vitro,* comprising the steps of recording at least two different spectra and/or spectral images of the tissue, analyzing the recorded spectra and/or spectral images by a multivariate data analysis to segment the tissues into distinct areas of similar spectral signature, and classifying each area as physiological, pathological or dead according to its spectral signature.

### Background of the invention

Each year several thousand patients in Germany are newly diagnosed for brain tumor. In about half of the cases, the brain tumor is a glioblastoma multiforme, which is known to be particularly malignant and to have a poor prognosis. Commonly, glioblastoma multiforme is treated by surgical resection followed by radiation and chemotherapy. The tumor should be resected as complete and accurate as possible to increase the patient's survival time, while preventing damaging vitally important brain areas. For example, resection of extended tumor tissue is limited where brain areas of memory, language, sensory or motor functions are concerned. However, due to the infiltrating growth of tumors in general and the microscopically vague demarcation of their borders, a precise and optimal resection is hardly possible, at present. Microscopically, the border between tumor tissue and healthy tissue is difficult to recognize, in particular because many cells are just at the transition from a normal to a pathological state. Usually, this transition can only be determined by molecular or genetic analysis.

Similar difficulties occur when resecting other kinds of diseased tissue, e.g. inflamed or dead tissue.

Therefore, methods are required to specifically identify and demarcate the diseased tissue from healthy surrounding tissue.

### Summary

The invention is directed to a method for a marker-free demarcation of distinct areas of a tissue *in vitro,* comprising the steps of recording at least two different spectra and/or spectral images of the tissue, analyzing the recorded spectra and/or spectral images by a multivariate data analysis to segment the tissue into distinct areas of similar spectral signature, and classifying each area as physiological, pathological or dead according to its spectral signature.

### Brief description of the drawings

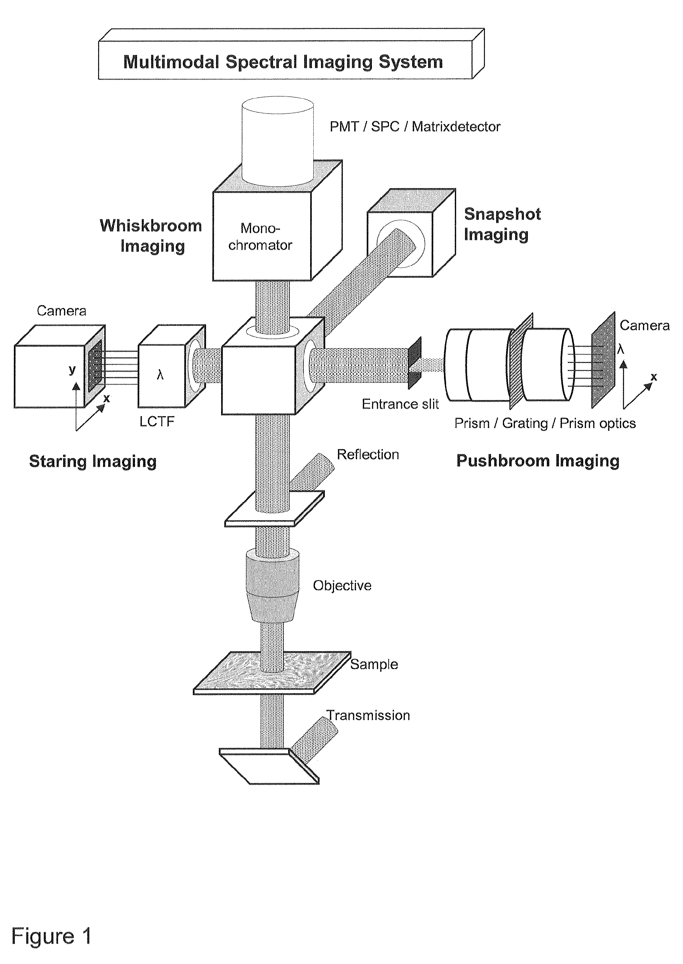

- Figure 1: shows an example of a multimodal spectral imaging system (MSIS) integrating various different spectroscopic techniques and measuring arrangements in a single unit. The MSIS comprises a detection unit consisting of a photomultiplier (PMT), a single photon counting (SPC), a matrix detector and a monochromator arranged for whisk-broom imaging, a camera and a monochromator like a liquid crystal tunable filter (LCTF), acousto-optical tunable filter or another wave-length tunable element arranged for staring imaging, and a camera with a dispersive optic like a prism/grating/prism arrangement for pushbroom imaging. A snapshot imager allows recording the spatial dimensions of the sample together with spectral properties at the same time without the need for scanning. Using the different arrangements simultaneously allows a marker-free characterization and demarcation of glioma tissue. The MSIS records spectra of different wavelength ranges using different spectroscopic techniques and different geometric arrangements.

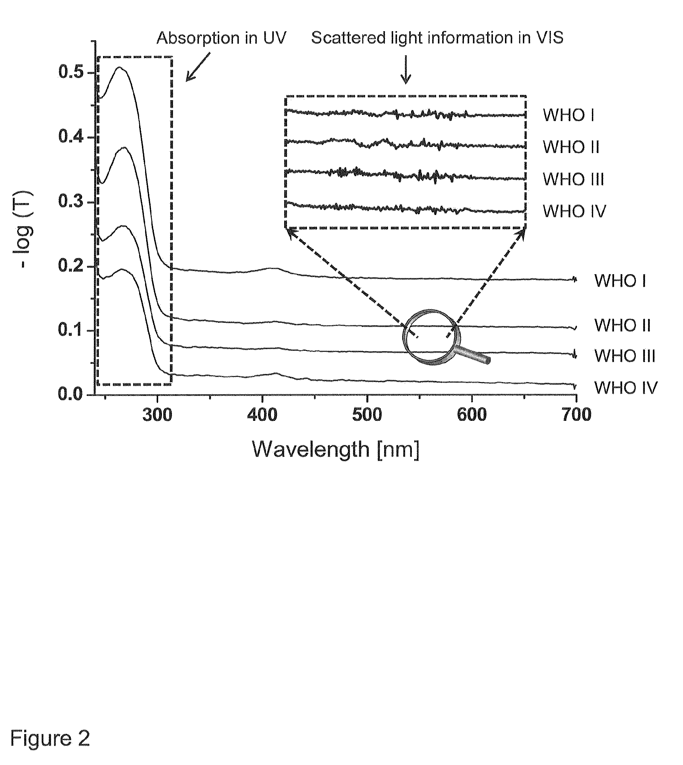

- Figure 2: shows UV/VIS spectra recorded in transmittance from glioma tissue. The mean spectra resolve the information from absorption and scattering spectrally with absorption occurring in the UV range and scattering occurring in the VIS range.

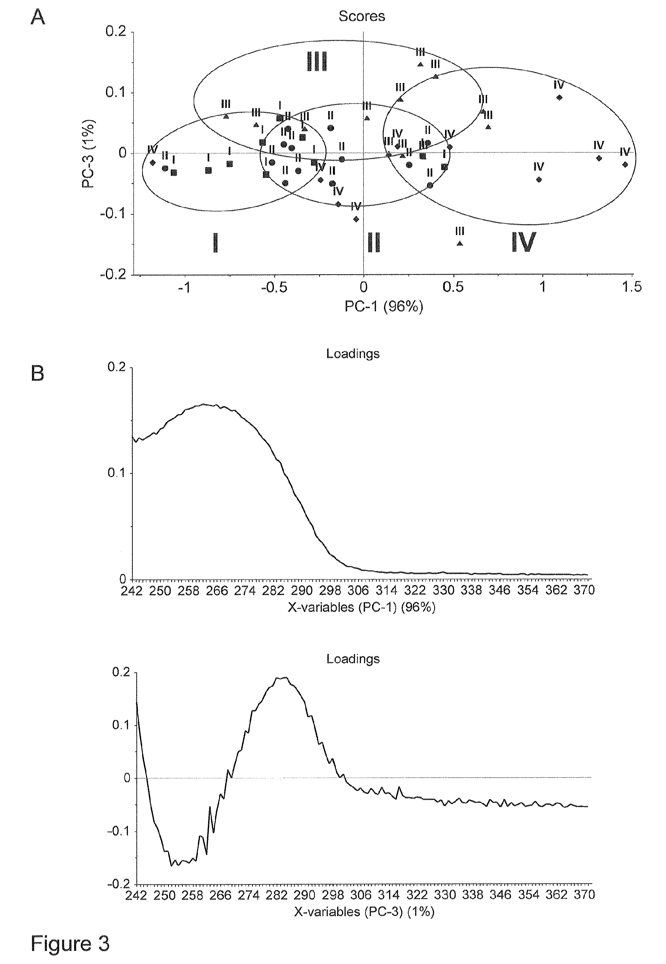

- Figure 3: shows the results of a principal component analysis (PCA) without preprocessing for the UV range of the spectra given in Figure 2(A). Additionally the loading plots are shown (B). The score plot of the PCA reveals a complete separation of tissue belonging to tumor grade WHO I and IV along the X-axis (principal component, PC1). Tissue of tumor grades WHO II and IIII overlap, but can be further distinguished by PC3 (Y-axis).

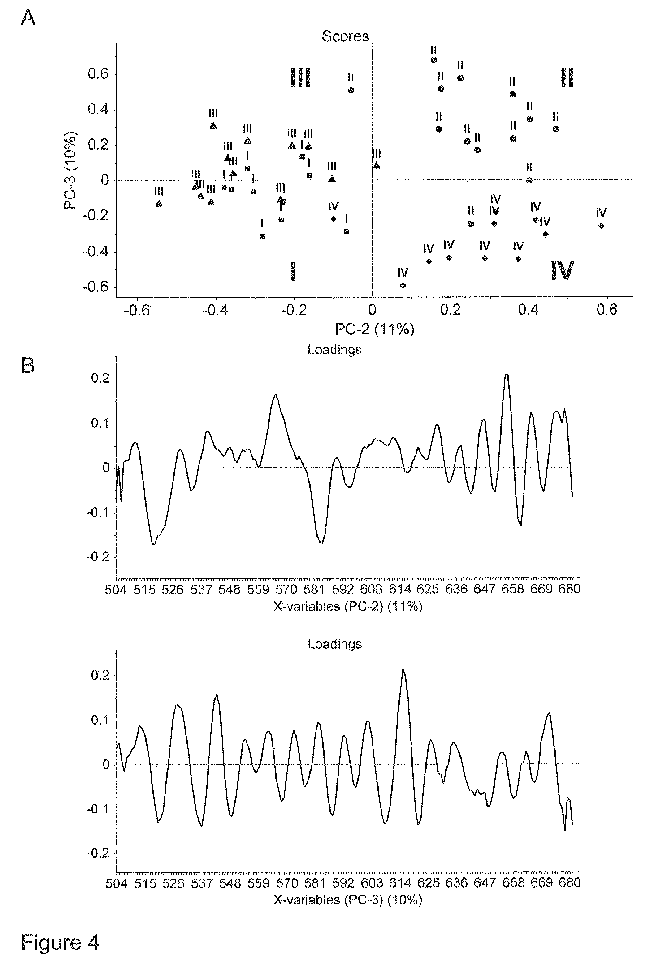

- Figure 4: shows a principal component analysis for the VIS range of the spectra given in Figure 2 comprising a preprocessing with smoothening (Savitzky-Golay, 2. order, 9 points), baseline corrections, and 1. derivative (Savitzky-Golay, 9 points) (A). Additionally the loading plot is shown (B). The score plot of the PCA of the VIS range shows a clear separation of tissue of tumor grades WHO I/III and WHO II/IV on the X-axis (principal component PC2). In addition, tissue of tumor grade WHO I and WHO III as well as tissue of tumor grade WHO II and WHO IV can be separated, respectively, by PC3 (Y-axis).

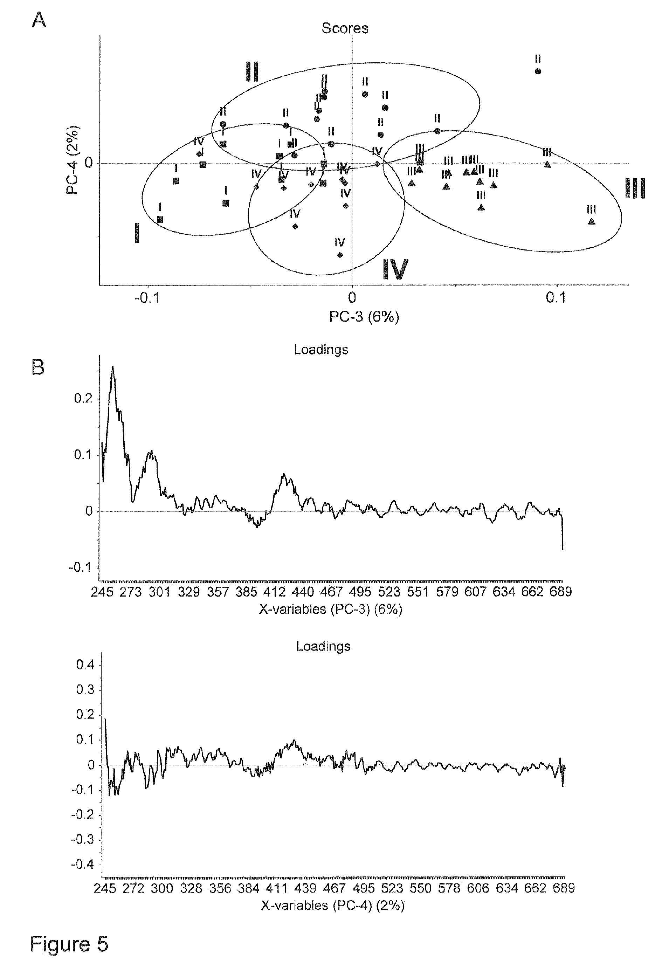

- Figure 5: shows a principal component analysis in the range of 245 to 700 nm, thus, covering the entire UV/VIS range (A). Additionally the loading plot is shown (B). The analysis was performed following preprocessing comprising smoothening (Savitzky-Golay, 2. order, 9 points), baseline offset, 1. derivative (Norris-GAP, 11 points), and normalized vector 1. The score plot of the PCA of the entire UV/VIS range shows a clear separation of tissue of tumor grades WHO I, III and IV by PC 3 (X-axis), whereas tissue of tumor grades WHO I, IV and II are further separated from WHO III by PC 4 (Y-axis).

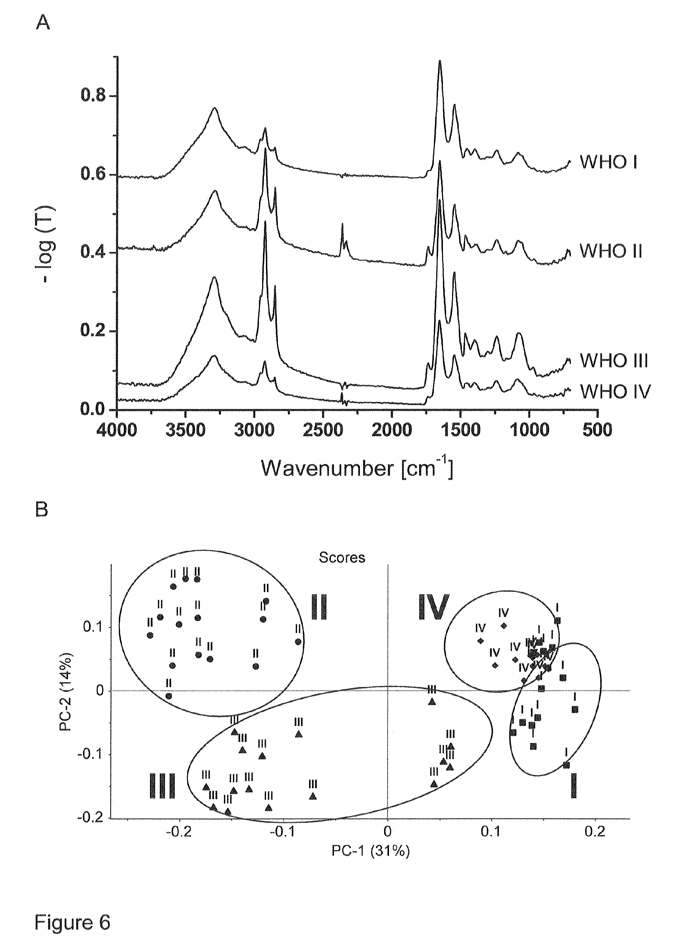

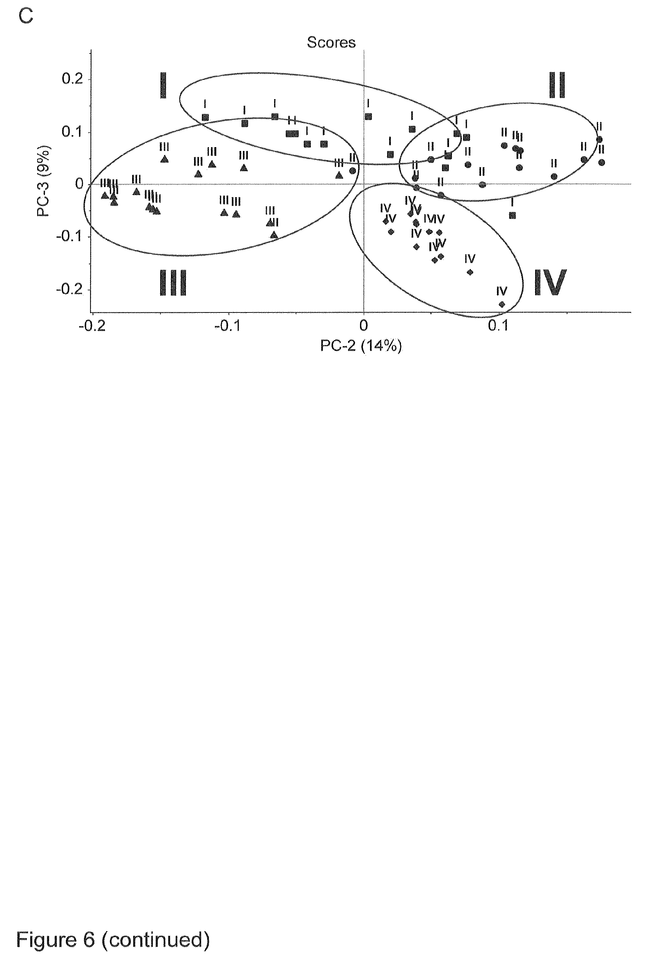

- Figure 6: shows infrared (IR) spectra recorded in transmission and their analysis. Mean spectra of glioma tissue comprising tissues of WHO grades I to IV show distinct absorption spectras for the four tumor grades (A). A principal component analysis of these spectra (range 2240 to 885 cm⁻¹) after preprocessing including smoothening (Savitzky-Golay, 2. order, 9 points), derivative (Norris-GAP, 9 points, 2. order) and normalized vector 1 are shown for PC 1 and PC 2 (B) and for PC 3 and PC 4 (C). The score plots show a specific separation of tissue of WHO grades I, II, III and IV considering PC-2 and PC-1 (B), as well as considering PC-3 and PC-2 (C).

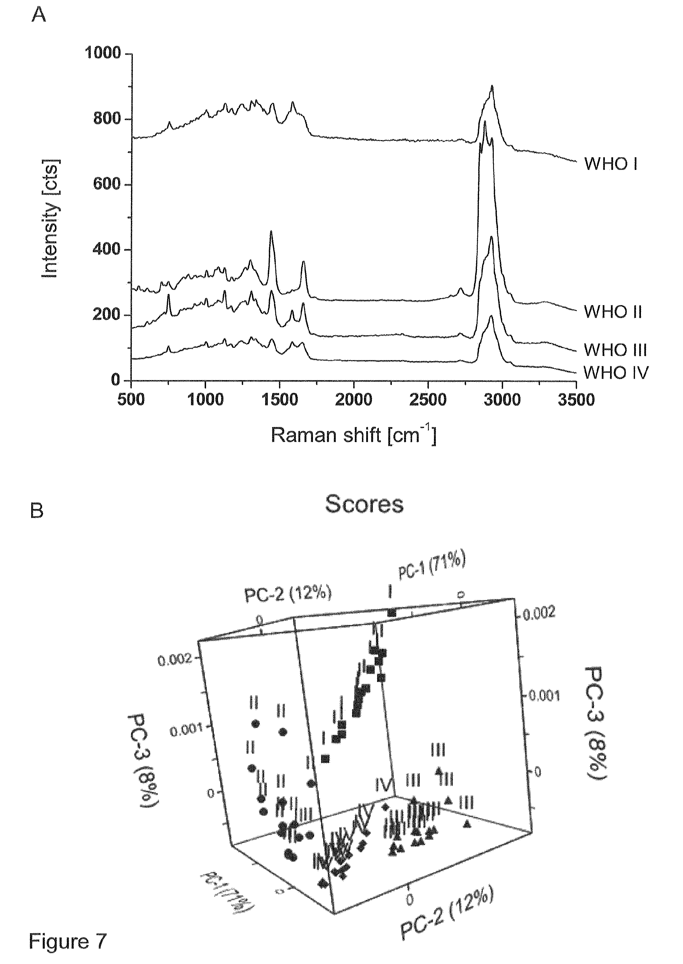

- Figure 7: shows Raman spectra of glioma tissue recorded in the reflection mode. Mean spectra (without preprocessing) recorded at an excitation of 532 nm reveals distinct Raman spectra for tissues of WHO grade I to IV (A). A principal component analysis comprising preprocessing (baseline offset), normalized vector 1, derivative (Savitzky-Golay, 2. order, 5 points) is shown in Figure 7B. The threedimensional scatter plot showing PC1, PC2 and PC3 depicts a distinct separation of tumor tissue of WHO grades I to IV.

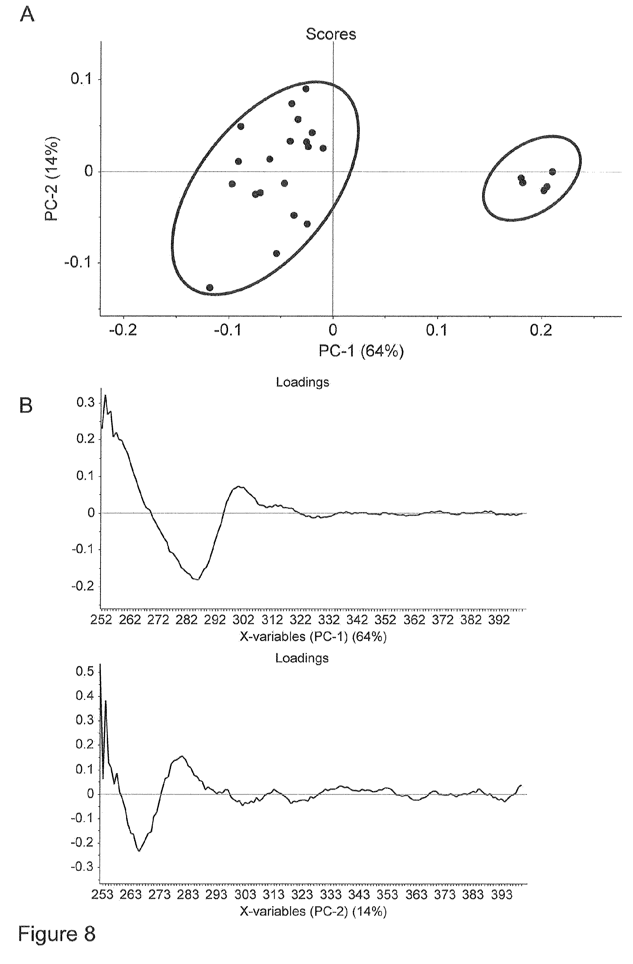

- Figure 8: shows a score plot of a principal component analysis of UV spectra acquired in the cell nuclei of glioblastoma cells with a specific part of apoptotic cells (A) and the corresponding loading plots for PC-1 and PC-2 (B).

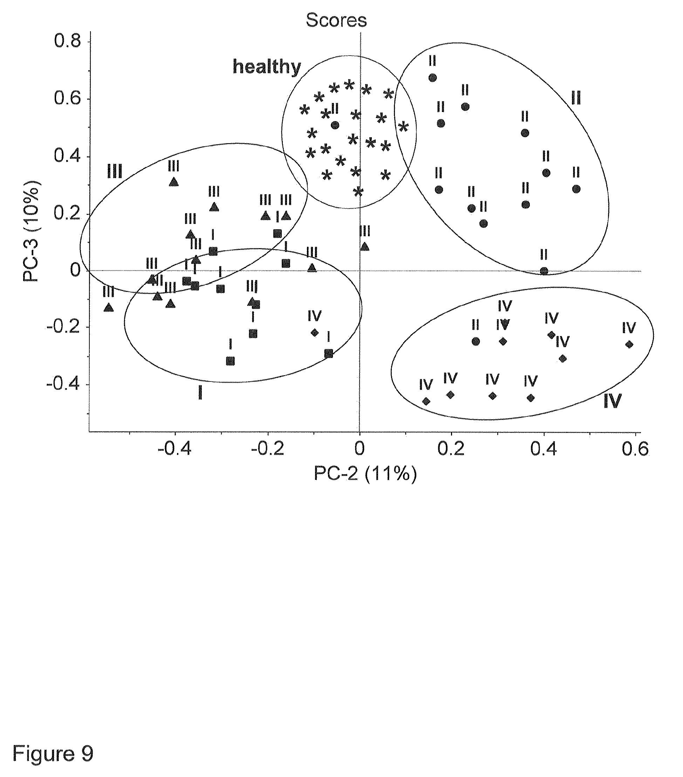

- Figure 9: shows a possible result for a principle component analysis of spectra recorded from a sample comprising healthy and tumor tissue including tissues of WHO grades I to IV. The score plot shows a specific separation of healthy tissue and tissue of WHO grades I, II, III and IV.

### Detailed description of the invention

The invention is directed to a method for a marker-free demarcation of distinct areas of a tissue *in vitro,* comprising the steps of recording at least two different spectra and/or spectral images of the tissue, analyzing the recorded spectra and/or spectral images by a multivariate data analysis to segment the tissue into distinct areas of similar spectral signature, and classifying each area as physiological, pathological or dead according to its spectral signature.

The term "marker-free" as used herein means without the need of introducing artificial markers. In particular, a marker-free demarcation of a distinct area of a tissue is achieved without introducing or applying artificial markers to the tissue prior to analyzing the tissue. Commonly, artificial markers such as dyes, fluorescent compounds or nuclear magnetic resonance (NMR) agents are used to label pathological tissues. These artificial markers can either be used as such or bound to antibodies or other molecules interacting with the pathological tissue, e.g. a tumor. However, artificial markers have distinct disadvantages as they are often not specific enough for a precise accumulation in tumor tissue only, which is essential for reliably demarking diseased tissue from physiological tissue. Moreover, many markers have to be taken prior to surgery and, thus, are an additional chemical burden to the patient. Finally, most of the commonly used markers rely on highly sophisticated equipment to be detected as e.g. NMR agents. The present method, in contrast, can be performed without the application of artificial markers.

The term "demarcation" as used herein refers to determining the boundaries of distinct areas within a tissue. This includes identifying the borders between areas of physiological cells, necrotic or apoptotic tissue as well as identifying the outline of an abnormal tissue, such as a tumor.

The term "spectrum" as used herein refers to the distribution of frequencies of electromagnetic radiation emitted, absorbed, reflected and/or transmitted by an object. A spectrum of an object may be generated by a series of recordings of radiation of different wavelength emitted, absorbed, reflected and/or transmitted by the object. Accordingly, the term "spectrum" includes emission spectra, absorption spectra and scattering spectra. The term "spectral image" as used herein refers to an image of a given object of at least two, preferably multiple wavelengths. The term includes e.g. multi-spectral images and hyper-spectral images. The spectral characteristics of a given object, e.g. a tissue, can be described as a series of spatially resolved spectra or as a series of spectrally resolved images, wherein each image corresponds to one wavelength. Accordingly, the present method can be based on spectra and/or spectral images recorded from the tissue to be analyzed.

The term "multivariate analysis" as used herein refers to statistic methods simultaneously processing and analyzing multiple parameter or variables in order to reveal their relationship and/or relevance. Multivariate analysis includes for example principal component analysis (PCA), factor analysis and multivariate analysis variance. Using a multivariate analysis, the spectra and/or spectral images of the tissue are characterized and areas having similar and different spectral signatures can be identified.

The term "spectral signature" as used herein refers to the spectral characteristics of an object expressed as the emission, absorption and/or scattering of radiation of or by the object. The spectral signature is individual for any given object or part thereof and, thus, can be used for the classification and identification of the object, or parts thereof.

The term "physiological" as used herein refers to living cells behaving according to their natural function and to tissue comprising, preferably consisting of, such cells. Physiological tissue or cells may also be referred to as "healthy" tissue or cells.

The term "pathological" as used herein refers to cells, which are altered such that they are no more able to fulfill their natural function and to tissue comprising, preferably consisting of, such cells. Pathological tissue may also be referred to as abnormal tissue, including e.g. inflamed tissue or tumor tissue of various tumor stages.

The term "dead" as used herein refers to necrotic tissue as well as to cells undergoing apoptosis.

The method of the invention enables identifying altered cells, in particular accumulations of pathological or dead cells, within a tissue or organ. Additionally, it is suitable to specifically determine the boundary between a physiological and pathological tissue. For example, the abnormal tissue is distinctly outlined, such that it can be precisely resected by surgery. The inventors found that physiological cells, pathological cells and dead cells each show different spectral characteristics. Although a single spectrum obtained from a tissue is not sufficient to directly identify areas of different health state within a complex tissue or organ, the inventors were able to achieve this by examining tissue using multimodal spectroscopy and analyzing the obtained data by a multivariate analysis. Thereby, distinct spectral signatures are revealed for areas of different physiological or pathological states within the tissue.

Using multimodal spectroscopy, at least two different spectra or spectral images are recorded from the tissue separately but simultaneously, i.e. at the same time, preferably by a multimodal imaging system. This provides pure spectra containing detailed and reliable information about the tissue, because different kinds of spectra and/or spectral images contain different information about the tissue. For example, absorption and scattered light spectra provide information on the chemical and morphological characteristics of the analyzed tissue, respectively.

Moreover, analyzing the obtained data by a multivariate data analysis results in a specific clustering of values for tissues of similar health state (see e.g. Figure 3 A). Thus, recording at least two different spectra and/or spectral images and analyzing them by a multivariate data analysis reveals specific spectral signatures that scientifically differ between physiological, pathological and dead tissue and, thus, allow a distinction of the different tissue areas. Based on this distinction, the areas are confined against each other and designated as physiological, pathological and dead. Thus, the tissue is segmented into areas of physiological, pathological and/or dead tissue. As a result, pathological or dead tissue can be demarcated within the otherwise healthy organ.

Knowing the exact boundary of abnormal or dead tissue and physiological tissue, respectively, enables the physician to completely remove the abnormal tissue without further destroying the remaining healthy organ. Importantly, the present method can be directly performed on a given tissue without the need of applying artificial markers, which makes the method particularly fast. To provide surgeons with information on the resected tissue, analyses have to be done during surgery, while the patient is still under anesthetics in case further tissue needs to be removed. Thus, pathological examination adds to the overall length of an operation. Using the present method, the tissue in question can be immediately analyzed without the need of adding any markers. In fact, the method is preferably performed directly on the resected tissue without the need of any preparation. e.g. fixation or sectioning. This significantly reduces the time of analysis and thus of the entire surgery. Given a sufficiently large operating room, the spectroscopic equipment may even be placed a long side the surgery table and the tissue analyzed immediately after resection.

For example, tumor tissue may be resected from the patient and analyzed using the method of the invention in order to reveal whether it comprises pathological and/or dead tissue only, or whether the boundary to the physiological tissue is already reached. If necessary, further tissue may be removed from the lesion and again analyzed until all tumor tissue is removed. Based on the information provided by the method of the invention, the surgeon can make sure to exclusively extend the resection in the direction of abnormal tissue.

In a preferred embodiment, the at least two different spectra and/or spectral images are selected from the group consisting of absorption spectrum, emission spectrum and scattered light spectrum. Each kind of spectrum is determined by specific characteristics of the object such that different kinds of spectra provide different information about the examined object. For example, absorption and emission spectra are determined by the atomic and molecular composition of an object. In contrast, scattered light spectra are determined by irregularities within an object (subsurface scattering) or on the object's surface (surface scattering). Although a spectrum is specific for the tissue it is recorded from, single spectra are not sufficient for identifying areas of different health state and for delimiting them from each other. However, the inventors found that by combining the information of at least two different kinds of spectra and/or spectral images, and by analyzing the obtained data using multivariate analysis, tissue areas of different health state can be distinguished and confined against each other.

In a particularly preferred embodiment, the at least two different spectra and/or spectral images comprise an absorption spectrum in combination with a scattered light spectrum. These two kinds of spectra can be recorded separately, however, at the same time, e.g. by using a multimodal imaging system. Thereby pure spectra can be obtained, based on which particularly specific and reliable spectroscopic signatures are generated. In detail, the absorption spectrum reveals the chemical characteristics and the scattered spectrum the morphological characteristics of the tissue.

In a preferred embodiment, the at least two different spectra and/or spectral images are recorded from different radiations and/or by different spectroscopic techniques. Spectra and spectral images can be recorded using various techniques. The imaging and recording techniques used in spectroscopy may be distinguished by the kind of radiation detected (e.g. UV/VIS, IR, Fluorescence, Raman), by the kind of measurement arrangements (e.g. reflection / backscattering, transmission or spatially offset) or by the kind of illumination (e.g. diffuse or normal incidence)..

The term "radiation" as used herein includes electromagnetic radiation of different wavelength ranges, e.g. UV/VIS, near infrared (NIR), and mid infrared (MIR), but also Raman scattering, fluorescence and photon diffusion. Accordingly, in a preferred embodiment of the invention, the radiation is selected from the group consisting of UV/VIS, near infrared (NIR), mid infrared (MIR), Fourier transformed infrared (FTIR), Raman scattering, fluorescence (including 2D fluorescence, auto-fluorescence and fluorescence including the time domain e.g. fluorescence lifetime spectroscopy and imaging, FLIM) and photon diffusion. Of these, UV/VIS and NIR are particularly preferred, since they can be recorded using rather easy to handle and low priced equipment. Moreover, they may be applied to any kind of tissue directly at the side of the surgery, since they do not need specific environmental conditions as e.g. in case of fluorescence, wherein absolute darkness is obligatory.

The term "spectroscopic technique" as used herein refers to methods for detecting specific interactions between radiation and matter. Accordingly, in a preferred embodiment, the setup for the spectroscopic technique is selected from the group consisting of transmission, reflection, attenuated total reflection, angular offset, laterally resolved offset and spatially offset, each possible under diffuse or specular illumination. Spatially offset is particularly preferred for fluorescence, Raman scattering and photon diffusion spectroscopy. Absorption spectra are preferably recorded in transmission or reflection, preferably under normal incidence, whereas diffuse incidence is also possible, providing chemical information. Scattering spectra are preferably recorded in transmission or reflection, preferably under diffuse incidence, whereas normal incidence is also possible, providing information on morphology.

In a preferred embodiment, one or more of the spectra and/or spectral images are recorded using whiskbroom imaging, pushbroom imaging, staring imaging or snapshot imaging. Spectroscopic imaging may be performed using different acquisition modi. When imaging an object using whiskbroom, also called mapping, each pixel of the object's surface is scanned and a complete spectrum recorded for each pixel. This provides detailed high resolution spectra for the entire tissue and significantly limits disturbance by photon diffusion. However, the method is slow for longer exposure times and generates large amounts of data, which need to be analyzed. Whiskbroom imaging is the preferred acquisition mode for IR and Raman spectroscopy. In pushbroom mode, the object is scanned by rows, wherein the detected light is resolved spectrally prior to recording. The pushbroom mode benefits from fast acquisition. Objects may move during acquisition in one direction.

In staring acquisition mode, 2-dimensional images of the entire object are taken for different wavelengths in sequence. With staring imaging, no spatial scanning of the sample or across the sample is performed. Instead, the wavelengths are scanned one by one. The light of defined wavelengths is isolated using monochromator elements like filters, liquid crystal or acousto-optical tunable filters or, in case of Fourier-transform techniques, using an interferometer. Depending on the monochromator element, a homogeneous illumination of the field of view is not easily achievable. Photon diffusion effects cannot be limited using this technique. However, its cost efficiency and the easy combination with standard microscopes or any other optical device with a camera port, are major advantages. Substrates with specific microstructure sizes are suitable for Mies scattering imaging with staring imaging. If the relevant wavelengths for an analytical problem are known, a fast spectroscopic analysis can be performed only based on these few wavelengths.

Unlike staring imaging, whiskbroom imaging or pushbroom imaging, snapshot acquisition does not involve scanning in spatial or spectral regard. Instead hyper-spectral images of an entire object are captured during a single integration time. Thus, spatial and spectroscopic information is collected simultaneously. This is a prerequisite for particular fast spectroscopic imaging, such that the technology is particularly advantageous for point-of-care testing devices.

In a preferred embodiment, the spectra are recorded with a spatial resolution of at least 10 µm, preferably at least 1 µm, more preferred at least 500 nm, most preferred up to 30 nm, which corresponds with a resolution up to near field. During surgery, abnormal or dead tissue should be removed as completely as possible, however, without damaging the surrounding healthy tissue, if possible. To achieve this, a high spatial resolution is beneficial for demarcating the pathological or dead tissue, since it enables drawing a discrete line outlining the tissue to be removed. For diagnostic purposes, a spatial resolution in the range of the size of about a cell nucleus (about 6 µm) or less is particularly advantageous.

In a preferred embodiment, the spectra and/or spectral images are pre-processed, preferably by smoothing, normalizations, derivatives, baseline corrections, mean centering, standard normal variate, de-trending, (extended) multiplicative scatter correction, orthogonal signal correction, science based filtering methods, and/or other applicable pre-processing methods. The pre-processing of the spectral information improves the accuracy and reliability of the spectral signature obtained by the multivariate analysis. In particular, smoothening, baseline corrections, normalizations and/or derivatives are best suited to improve the analysis of spectra and/or spectral images recorded for the UV/VIS range and efficiently distinguish the different classes of tumor tissue.

In a preferred embodiment, the multivariate data analysis comprises at least one methodology selected from the group consisting of principal component analysis (PCA), partial least squares regression for clustering and discrimination and self independent modeling of class analogies (SIMCA), which are the most used methods, but also techniques like multivariate analysis of variance (MANOVA), canonical correlation analysis, redundancy analysis (RDA), correspondence analysis (CA), canonical (or constrained) correspondence analysis (CCA), multidimensional scaling, principal coordinates analysis (PCoA; based on PCA), discriminant analysis, linear discriminant analysis (LDA) clustering systems, recursive partitioning, artificial neural networks and support vector machines (SVM). Multivariate curve resolution (MCR) and other resolution algorithms, as e.g. science based calibration (SBC, a so called filter method), allow integrating first principle knowledge from other sources, which improves the robustness of the prediction. The inventors found that by recording two different spectra and/or spectral images and analyzing them by a multivariate data analysis, spectral signatures are provided that differ considerably for tissues of different health states. The recorded spectra and/or spectral images comprise large amounts of data, which, although individual for each tissue, are not sufficient to distinguish specific areas within the tissue in respect to their health state. However, by subjecting the data to a multivariate analysis specific data points and values can be extracted for different areas, which represent the areas' spectral signatures. The spectral signatures were found to specifically correlate with the health state of a tissue or area therein. For example, spectral signatures of tumor tissue of WHO grades I to IV cluster together, respectively. Likewise spectral signatures of necrotic tissue cluster together while distinctly separating form spectral signatures of physiological tissue. Thus, analyzing the spectra and/or spectral images using multivariate analysis allows for classifying each area of a given tissue by designating the area as physiological, pathological or dead. Moreover, when analyzing UV/VIS spectra or spectral images, the principal component analysis, preferably of two or three principal components, was found to be particularly suitable. Likewise, combining the principal component analysis with pre-processing by smoothening, normalization, derivatives and/or baseline correction was found to provide particularly detailed and reliable data analysis and clustering, which are robust. The obtained spectral signatures were sufficient to distinguish tissues belonging to WHO tumor grades I, II, III or IV.

In a preferred embodiment, the tissue is not pre-treated by preservation and/or slicing. Commonly, tissue analysis is based on the use of markers that interact with pathological or dead tissue but not with physiological tissue. These markers have to be brought into contact with the tissue to be analyzed, such that they can penetrate into the tissue. To achieve this, the tissue is sectioned, usually following fixation, which takes considerable time. Since such procedures are likewise necessary for simple hematoxilin and eosin stainings as for fluorescently coupled antibodies and other more sophisticated detection methods, sectioning is obligatory once markers have to be used. Alternatively, the marker may be administered to the patient prior to surgery, such that it has already accumulated in the pathological tissue at the time of the resection. However, for detection of the marker, in most cases, the resected tissue has to be sectioned, too. In contrast, the described method is exclusively based on analyzing the raw surface of a tissue, i.e. without the use of markers. The spectroscopic analysis is performed immediately on the resected tissue without the requirement of preservation as e.g. fixation or sectioning. Moreover, to reveal the extension of the abnormal tissue, in particular to determine whether the border between the pathological and the physiological tissue has already been reached, the resected tissue may be analyzed from all sides. In case the spectroscopic equipment can be localized next to the side of surgery, any transfer of the tissue, e.g. to a pathology department, can be avoided rendering even any conservation of the tissue moot. All this makes the method particularly fast, which is of significance, since the duration of a surgery is often defined by the time for analyzing the resected tissue. Thus, by reducing the time of analysis, the patient's burden by the length of the surgery can be reduced improving his probability for recovery. Additionally, it is not necessary to administer any markers to the patient prior to surgery, further reducing the burden on the patient's health.

In a preferred embodiment, the tissue is cancerous tissue, inflamed tissue, apoptotic tissue, and/or necrotic tissue. By recording and analyzing at least two different spectra and/or spectral images distinct spectral signatures can be obtained that differ for tissues of different health state. This enables a demarcation of inflamed tissue, cancerous tissue, apoptotic tissue as well as necrotic tissue from the neighboring physiological tissue.

In a preferred embodiment, the tissue is derived from brain, skin, intestine, cervix, liver, ovar, gall bladder, oropharynx, oesophagus, tongue or bladder.

In a preferred embodiment, the tissue is cancerous tissue and the cancer is selected from the group consisting of brain tumor, astrocytic tumor (astrocytoma (WHO grade II), anaplastic (malignant) astrocytoma (WHO grade III), glioblastoma multiforme (WHO grade IV), pilocytic astrocytoma (non-invasive, WHO grade I), subependymal giant cell astrocytoma (non-invasive, WHO grade I), pleomorphic xanthoastrocytoma (non-invasive, WHO grade I), oligodendro-glial tumor (oligodendroglioma (WHO grade II), anaplastic (malignant) oligodendroglioma (WHO grade III)), ependymal cell tumor (ependymoma (WHO grade II), anaplastic ependymoma (WHO grade III), myxopapillary ependymoma, subependymoma (WHO grade I)), oligoastrocytoma (WHO grade II), astroblastoma (WHO grade IV), schwannoma and neurofibroma.

In a preferred embodiment, the tissue is cancerous tissue and the areas are classified with respect to their state of malignancy, preferably according to WHO classification of tumors, more preferred distinguishing WHO grades I, II, III and IV. During transformation of a physiological tissue to cancerous tissue, the cells undergo stepwise transformation due to or accompanied by the accumulation of genetic defects. Accordingly, tumor cells and tissue can be distinguished by their state of transformation. For tumors of the central nervous system, for example, the WHO classification has been implicated, which distinguishes tumor grades I to IV. Within this system, grade I refers to slow-growing, non-malignant tumors that are in general associated with long-term survival of the patient. Depending on their form and position they may be even cured by total resection. Grade II tumors are rather slow-growing, malignant or non-malignant tumors, which, however, can recur as higher grade tumors. Grade III tumors are malignant and often recur as higher grade tumors, namely as grade IV tumors, which are rapidly growing and very aggressive malignant tumors and are, thus, associated with severely reduced survival times. Commonly, grade IV tumors are, for example, glioblastoma and medulloblastoma. Despite these distinct tumor states, tumor sites can comprise cells of several different grades. This is of importance for tumor surgery, since the total removal of grade III and IV cells is crucial, whereas cells or areas of tissue of tumor grades I or II may be maintained, in case they concern tissue that is located within or close to a vital tissue or organs. Additionally, in case the surgery aims at a complete removal of the tumor tissue, a reliable distinction between cells of the tumor, in particular grade I and II from healthy tissue is essential. An exclusively visual distinction of these cells is hardly ever possible, even using light microscopy, because the cellular transformation of grade I or grade II is subtle and can in most cases only be determined by genetic or molecular analysis. Here, the method of the invention provides an alternative approach for immediately identifying such tissue without the need of time consuming cytological or molecular examination. The inventors found that the molecular and genetic alterations occurring during tumor progression are reflected in the spectral properties of the cells. These can be captured using the methods describes herein. Thus, the method of the invention allows a demarcation of areas of different tumor grades enabling the surgeon to specifically decide which areas of tissue to remove.

Further described is a method for a marker-free demarcation of distinct areas of a tissue comprising the steps of recording at least two different spectra and/or spectral images of the tissue, analyzing the recorded spectra and/or spectral images by a multivariate data analysis to segment the tissue into distinct areas of similar spectral signature, and classifying each area as physiological, pathological or dead according to its spectral signature. This method may also be performed *in vivo.* Since the method is based on the direct spectroscopic analysis of a tissue's surface, namely without the use of any exogenous marker, it can be applied directly to the patient. For example, a surgeon may expose an organ or tissue known to comprise a tumor. The surface of the organ or tissue is then examined by recording at least two different spectra and/or spectral images which are further analyzed by a multivariate data analysis. The generated spectral signatures are used to classify areas of the tissue as physiological, pathological or dead. In addition they allow establishing the boundaries between said areas throughout the tissue or organ. Thereby, the outline of a pathological tissue, e.g. a tumor, can be determined prior to resection avoiding removing any healthy and functional tissue. The surgeon can directly decide on the extent of the resection. If necessary, the surgeon may remove tissue in layers, each time analyzing the organ's surface for determining the lateral extension of the pathological tissue before removing the next layer.

Further described is a method for determining a resection border comprising the steps of recording at least two different spectra and/or spectral images of a tissue to be resected, analyzing the recorded spectra and/or spectral images by a multivariate analysis to identify different areas of similar spectral signature within the tissue, classifying each area as physiological, pathological or dead according to its spectral signature, and generating a map of the tissue indicating the border along the different areas. The method allows for identifying the health state of different areas of a given tissue and to determine the borders between these different areas. From the recorded and analyzed spectra and/or spectral images a map of the analyzed tissue can be generated, wherein the distinct areas of different health state and their borders are indicated. The map is provided to the surgeon such that he may resect pathological tissue along the indicated borders. The method is particularly advantageous because it allows for distinguishing different states of pathological or dead tissue. In particular, the method is suitable for distinguishing tumor cells of different tumor grades as well as apoptotic and necrotic tissue. Knowing the exact localization and identity of tumor cells, the surgeon is provided with more comprehensive and meaningful information to base his decision for resection onto. Using common methods, the specific health state of individual areas of a pathological tissue can hardly be determined during a surgery. For example, comprehensive genetic and molecular examinations take too long to be performed while the patient is still under surgery. As a result any molecular or genetic examination has to be done either on tissue removed by a biopsy prior to the surgery or on the resected tissue after the surgery is terminated. Unfortunately, biopsies only provide a small and random sample of the pathological tissue. In contrast, analysis of the resected tissue may be comprehensive, but in case of an unexpected result may require a second surgery to ensure that the complete tumor tissue is removed. In this respect, the present invention provides a distinct advantage over both strategies, as the characteristics of the pathological tissue, e.g. the tumor, can be determined during surgery or in case of a direct analysis, even before resection. This significantly improves the quality of surgery, increasing the accuracy of resection and reducing the risk of unnecessarily damaging functional and healthy tissue.

In a preferred embodiment, the spectra are recorded using a multimodal spectral imaging system. The multimodal spectral imaging system may comprise a spectrometer, a spectrograph, at least one objective, at least one camera, optics, optical fibers, optical filters, (matrix) detector, pushbroom imager, illumination, light source, control electronics and software, multivariate toolbox, laser, a staring imager, a snapshot imager, a camera combined with a liquid crystal tunable filter (LCTF) (Staring imaging), a camera combined with a prism-grating-prism (pushbroom imaging), a photomultiplier tube combined with a monochromator and/or a single photon counting module combined with a monochromator (whiskbroom imaging).

In a preferred embodiment the tissue to be resected is a tissue comprising a tumor, preferably comprising a brain tumor.

In a preferred embodiment, classifying each area as pathological comprises distinguishing areas of different tumor grade, preferably of WHO tumor grades I, II, III and IV.

### Examples

### 1. Multimodal spectral imaging system

A multimodal spectral imaging system (MSIS) integrates several different spectroscopic techniques and measurement arrangements in a single unit **(****Figure 1****).** The MSIS comprises in this example an objective for observing a sample and several devices for recording spectra and/or spectral images from the sample. The objective-less approach could be alternatively applied. The setup may comprise a monochromator and a photo multiplier and/or a single photon counting and/or a matrix detector adapted for whiskbroom imaging, a camera and a liquid crystal tunable filter arranged for staring imaging in combination with a camera connected to pushbromm imaging optics (e.g. realized by a prism/grating/prism arrangement). A snapshot spectral imager can be used for fast multidimensional spectroscopic imaging without the need for spatial or spectral scanning. By integrating these various systems a marker-free characterization of a sample, e.g. a tissue, organ or part thereof, is possible. The MSIS records spectra of different wavelength ranges using diverse spectroscopic techniques and measurement arrangements. This allows the recording of distinct spectra, e.g. absorption spectra, scattering light spectra and fluorescence spectra from a single sample simultaneously.

### 2. Analyzed tissue

Samples analyzed by multimodal spectroscopy, included long term cultures of malignant glioma cells, explant cultures from tumor tissue and freshly resected and nitrogen frozen tumor tissue. For evaluating the reproducibility of spectroscopic recordings and for comparing recordings from explant cultures and frozen tissues, spectroscopic signatures were obtained from various samples and compared to each other. Scattered light spectra and absorption spectra were recorded in reflection or transmission for the UVNIS and near infrared range. These data revealed reliable spectral signatures for frozen tissue as well as for explant cultures.

Tissue of brain tumors was frozen in liquid nitrogen immediately after excision without the use of Ringer's solution.

The samples were analyzed using microscope slides made from quartz glass, (Heraeus Suprasil 1, Germany), gold coated microscope slides (Erie Scientific, ThermoFisher Scientific, USA) or BaF₂ microscope slides, which were preferably used for infrared and Raman spectroscopy.

Spectra and spectral images were recorded following quick sectioning but may be recorded directly from the tissue, as well. Fixation of the tissue using e.g. paraformaldehyde is possible but not necessary.

Alternatively, the tissue may be analyzed *in situ* by an optical access to the surgical site e.g. using a operation microscope or endoscope. The surgical microscope may be used as an optical sensor. Thereby, light is directed via a specific optical path to the multimodal spectral imaging system for recording the spectra or spectral images. Illumination of the tissue may likewise be achieved via the multimodal spectral imaging system. Using a microscope or an endoscope, the MSIS may be connected to the surgical tool via optical light fibers or any other light delivery equipment, e.g. mirrors, lenses or GRIN lenses.

### 3. Recording spectra, data analysis and classification of tumor samples

Samples of tumor tissue were characterized by recording absorption and scattered light spectra in the UV/VIS and infrared range and using Raman spectroscopy and 2D auto-fluorescence spectroscopy.

The recorded spectra were analyzed using principal component analysis and multivariate methods, which revealed a correlation between the obtained spectra and the malignancy of the tumor tissues as e.g. known from magnetic resonance tomography, classical pathological examination and fluorescence markers such as 5-ALA. By separating the obtained spectra or spectral images into their basic spectra and calculating the principal components of the obtained data, a specific correlation of distinct spectral signatures for different types of tumor tissue was revealed.

Recording absorption spectra in the VIS range in transmission or reflection under normal light incidence provided spectral information based on the chemical and molecular characteristics of the tissue. In addition, scattering light spectra obtained in dark field diffuse reflection provided spectral information on the morphology of the tissue. Separating the obtained absorption and scattering light spectra spectrally revealed a distinct absorption pattern in the UV range and scattered light spectrum in the VIS range for tissues of different tumor grades **(****Figure 2****).** Analyzing this data using a principal component analysis for the range of 240 to 370 nm revealed a distinct and separate clustering of spectral signatures obtained from tissue of tumor Grade I and obtained from tissue of tumor Grade IV **(****Figure 3****).**

Analyzing the same data using a principal component analysis for the range of 500 to 685 nm including preprocessing of smoothening (Savitzky-Golay, 2. order, 9 points), first derivative (Norris-gap, 9 points, 2. order) and vector 1 normalization resulted in a distinct clustering of spectral signatures obtained from tumor tissues of Grades I and III and of tissues obtained from tumor Grades II and IV, respectively **(****Figure 4****).**

Additionally, the entire data of the wavelength range of 245 to 700 nm was analyzed by a principal component analysis in combination with a preprocessing comprising smoothening (Savitzky-Golay, 2. order, 9 points), baseline offset, first derivative (Norris-GAP, 11 points) and unit vector normalization. This resulted in a distinct clustering of spectral signatures obtained from tissues of tumor Grade I, II, III and IV. Compared to the analysis of distinct spectral ranges (compare **Figure 3** and **Figure 4**), the clustering shows a different arrangement due to a shift in the main emphasize of the data points which resulted from jointly considering absorption and scattering spectra.

Score plots of a principal component analysis based on spectra recorded in the infrared range (2240 to 885 cm⁻¹) also revealed a distinct separation of the spectral signatures obtained from tissue of tumor grades WHO I, II, III and IV **(****Figure 6****).**

A similar distinction of tissues of different tumor grades was achieved based on the analysis of Raman spectra **(****Figure 7****).**

### 4. Recording spectra, data analysis and classification of apoptotic and necrotic samples

Apoptosis, also referred to as programmed cell death, plays a major role for a broad variety of cellular processes. These include not only developmentally physiological procedures but also processes in the adult organism like the removal of abnormal cells, the control of the cell number and the size of tissues or the degradation of harmful cells. During apoptosis the cell shrinks and organelles and the membrane condense. Constrictions of the membrane take place, apoptotic bodies are generated and DNA is degraded. In an advanced stage of apoptosis the cells bloat. The various stages of apoptosis can be observed using spectral imaging. For example, cell nuclei and the compartments around the nuclei are well visible without staining under dark field illumination. Thus, the dark field mode is applied for spectroscopic characterization of apoptotic cells.

For example, during the cultivation of glioblastoma cells from freshly removed explants, apoptosis is induced using a cytostatic drug. UV mean spectra of the nuclei of the glioblastoma cells were then recorded in transmission, primarily providing chemical information, followed by a principal component analysis of the spectral data (**Figure 8** **A,** loading plots of the PC-1 and PC_2 are given in **Figure 8** **B**). The formation of two clusters was found. The big cluster includes 19 measurements, the small cluster 5 measurements. By that, 21 % of all measured cells are classified as different. The characterization of the apoptotic cells was further improved by combination with further spectroscopic techniques like scattered light spectroscopy in the dark field, which offers additional morphological information, and IR spectroscopy providing vibrational spectroscopic information on the molecular level.

Necrosis refers to a different type of cell death, which in contrast to apoptosis is not a physiological process. The death of single cells or cell populations is triggered by detrimental influences like bacteria, toxins or a lack of nutrients or oxygen. For the pathological grading of tumor tissues according the WHO scale, the presence of necrosis is one of four important parameters beside cell density, pleomorphism and mitosis. In this way the tumors may be assigned to low-grade (WHOI-II) and higher-grade tumors (WHO III-IV). Due to the cellular alterations occurring specifically during necrosis, necrotic tissue may be distinguished from apoptotic tissue using a multimodal spectroscopic imaging approach.

### 5. Recording spectra, data analysis and classification of tumor samples in comparison to physiological tissue

Physiological tissue does not show the tumor specific characteristics of deviant cell density, pleomorphism, mitosis and/or necrosis. Accordingly, physiological tissue and tumor tissue differ in their chemistry as well as morphology. These alterations in chemistry and morphology are accessible via the multimodal microspectroscopic imaging approach.

Multimodal microspectroscopic measurements and data analysis are performed as described above on tissue comprising physiological cells and tumor cells. This reveals that the data obtained from physiological tissue forms a cluster distinct from the clusters obtained from the spectroscopic data of tumor tissue (Figure 9). Although, depending on the measurements performed and the analysis methods used, the position of the cluster of the physiological tissue will vary with respect to the clusters of the tumor tissue, it can be clearly distinguished due to the different spectroscopic properties of the various tissues.

## Claims

1. A method for a marker-free demarcation of distinct areas of a tissue *in vitro,* comprising the steps of
- recording at least two different spectra and/or spectral images of the tissue,
- analyzing the recorded spectra and/or spectral images by a multivariate data analysis to segment the tissue into distinct areas of similar spectral signature, and
- classifying each area as physiological, pathological or dead according to its spectral signature.

2. The method according to claim 1, wherein the at least two different spectra and/or spectral images are selected from the group consisting of absorption spectrum, emission spectrum and scattered light spectrum.

3. The method according to claim 1 or 2, wherein the at least two different spectra and/or spectral images comprise an absorption spectrum in combination with a scattered light spectrum.

4. The method according to any of claims 1 to 3, wherein the at least two different spectra and/or spectral images are recorded from different radiations and/or by different spectroscopic techniques.

5. The method according to claim 4, wherein the radiation is selected from the group consisting of UV/VIS, near infrared (NIR), mid infrared (MIR), Fourier transformed infrared (FTIR), Raman scattering, fluorescence and photon diffusion.

6. The method according to claim 3 or 4, wherein a setup for the spectroscopic technique is selected from the group consisting of transmission, reflection, attenuated total reflection, angular offset, laterally resolved offset and spatially offset, each under diffuse or specular illumination.

7. The method according to any of claims 1 to 6, wherein one or more of the spectra or spectral images are recorded using whiskbroom imaging, pushbroom imaging, staring imaging or snapshot imaging.

8. The method according to any of claims 1 to 7, wherein the spectra are recorded with a spatial resolution of at least 10 µm, preferably at least 1 µm, more preferred at least 500 nm, most preferred up to 30 nm.

9. The method according to any of claims 1 to 8, wherein the recorded spectra and/or spectral images are pre-processed, preferably by smoothing, normalizations, derivatives, baseline corrections, mean centering, standard normal variate, de-trending, multiplicative scatter correction, orthogonal signal correction, and/or science based filtering methods.

10. The method according to any of claims 1 to 9, wherein the multivariate data analysis comprises at least one methodology selected from the group consisting of principal component analysis (PCA), partial least squares regression for clustering and discrimination, together With SIMCA, multivariate analysis of variance (MANOVA), factor analysis, canonical correlation analysis, redundancy analysis (RDA), correspondence analysis (CA), canonical correspondence analysis (CCA), multidimensional scaling, principal coordinates analysis (PCoA; based on PCA), discriminant analysis, linear discriminant analysis (LDA) clustering systems, recursive partitioning, artificial neural networks and support vector machines.

11. The method according to any of claims 1 to 10, wherein the tissue is not pre-treated by preservation and/or slicing.

12. The method according to any of claims 1 to 11, wherein the tissue is cancerous tissue, inflamed tissue, apoptotic tissue, and/or necrotic tissue.

13. The method according to any of claims 1 to 12, wherein the tissue is derived from brain, skin, intestine, cervix, liver, ovar, gall bladder, oropharynx, oesophagus, tongue or bladder.

14. The method according to claim 12 or 13, wherein the tissue is cancerous tissue and the areas are classified with respect to their state of malignancy, preferably according to WHO classification of tumors, more preferred distinguishing WHO grades I, II, III and IV.
